# DEMANDE DE BREVET EUROPEEN

(11) **EP 2 213 263 A1**
(43) Date de publication de la demande: **04.08.2010**
(21) Numéro de dépôt: 10151777.9
(22) Date de dépôt: 27.01.2010
(51) Int. Cl.: A61F 2/42, A61B 17/56

(54) **Implant pour la correction du pied plat**

(30) Priorité: 30.01.2009 FR 0950603
(71) Demandeur: Textile Hi-Tec (T.H.T.), 34220 Verreries de Moussans (FR)
(72) Inventeur: Houard, William, 81270, LABASTIDE ROUAIROUX (FR)
(74) Mandataire: Cochonneau, Olivier

(57) **Abrégé**

L'implant (1) pour la correction du pied plat comprend :
- un corps central (5) avec des parties distale (5a) cylindrique, proximale (5b) tronconique et intermédiaire (5c), celle-ci comportant des moyens externes de déplacement (7),
- une enveloppe externe (6), de forme cylindrique, avec des parties distale (6a) et proximale (6b) comportant des fentes longitudinales (10, 12) définissant une pluralité d'ailettes distales (11) et proximales (13) et une partie intermédiaire (6c) comportant des moyens internes de déplacement (15), et dont le diamètre (D2) est supérieur à E.

La face intérieure (11a) des ailettes distales (11) est inclinée vers l'axe longitudinal (XX') de l'enveloppe externe (6).

La face intérieure (13a) des ailettes proximales (13) est parallèle à l'axe longitudinal (XX').

La partie intermédiaire (6c) étant insérée dans la zone de dimension minimale E du sinus (4) du tarse, le déplacement du corps central (5) à l'intérieur de l'enveloppe (6) provoque le soulèvement des ailettes proximales (11) et distales (13).

## Description

La présente invention concerne un implant ou endorthèse destinée à reformer la voute plantaire naturelle dans la pathologie dite du pied plat.

La déformation de la voûte ou arche plantaire, dans la pathologie du pied plat, est due principalement à un défaut d'alignement du talus ou astragale et du calcanéum ou calcaneus. Pour pallier ce défaut d'alignement, il est connu d'introduire un implant ou endorthèse dans le sinus du tarse, qui est le canal s'étendant entre le talus et le calcanéum au niveau des surfaces articulaires antérieure et postérieure existant entre ces deux os.

Le document W0.2005/081704 décrit un implant qui comporte une partie distale cylindrique présentant un filetage externe, relié à une partie proximale de configuration tronconique comportant éventuellement un prolongement cylindrique. L'introduction de cet implant dans le sinus du tarse se fait par vissage, le filetage externe de la partie distale s'engageant de manière active dans les tissus osseux environnant en sorte d'assurer le déplacement et l'ancrage de l'implant dans la zone de dimension minimale du sinus du tarse. Quant à la partie proximale tronconique et l'éventuel prolongement cylindrique, ils sont censés former des appuis localisés sur le talus et le calcanéum dans la zone d'entrée du sinus du tarse. Ainsi, dans ce document, la stabilisation de l'implant est obtenue par le filetage externe de la partie distale et un éventuel rainurage formé dans le prolongement cylindrique, ce filetage et ce rainurage peuvent être le siège de fibrose empêchant ou rendant difficile le retrait ultérieur de l'implant.

Le document EP.0.560.249 décrit un implant qui est du type expansif. Il comprend un premier corps cylindrique terminé par une tête semi-sphérique. Ce corps comprend un trou borne axial avec une partie cylindrique fileté et une partie légèrement conique décroissant en diamètre depuis la partie filetée vers la tête semi-sphérique. Une incision axiale à travers la tête semi-cylindrique et se prolongeant vers la partie filetée définit deux battants déformables.

L'implant comporte également un second corps cylindrique dont la partie distale filetée est apte à coopérer avec la partie filetée interne du premier corps. Pour la mise en place de l'implant, le premier corps est introduit dans le sinus du tarse puis le second corps est vissé à l'intérieur du premier corps. Lors de ce vissage, le second corps s'engage avec la portion conique du trou borgne axial du premier corps, déformant et écartant vers l'extérieur les deux battants de façon à les amener en contact avec le talus et le calcanéum. C'est donc l'expansion des deux battants qui définit l'écartement du talus et du calcanéum qui est souhaitée pour la correction du pied plat. La stabilisation de l'implant est obtenue par des rainures annulaires qui définissent entre elles de petits espaces pour la croissance du tissu fibreux entre l'implant et les os attenants.

D'autres documents décrivent des implants expansifs du même type. Notamment les documents US 2008/0208349A1, EP.1.728.491 et EP 983.755.

Le document EP 1.728.491 prévoit des moyens de blocage anti-retour destinés à empêcher l'expulsion de l'organe interne qui réalise l'expansion transversale de l'enveloppe externe. Dans ce document la stabilisation de l'implant est réalisée grâce à la présence d'ailettes qui sont ménagées sur la surface externe de l'enveloppe expansible, lesdites ailettes s'opposant à l'expulsion de l'implant après sa mise en place et son expansion au sein de la cavité du sinus tarsien.

Dans le document EP.983.755, l'implant est composé de trois parties, à savoir un corps central cylindrique fileté comportant une tête de blocage en translation, un cône d'expansion dont le filetage interne à sa partie proximale est apte à coopérer avec le filetage du corps central et une couronne externe qui peut être expansée par le cône d'expansion lors du vissage du corps central et son déplacement corrélatif dans la couronne externe depuis la partie distale vers la partie proximale. Dans ce document, la stabilisation de l'implant est obtenue grâce à la présence sur la face externe de la couronne d'ailettes qui ont pour fonction de s'opposer à l'expulsion de l'implant une fois que celui-ci a été mis en place. Cependant, ces ailettes, comme un filetage ou un rainurage, peuvent être le siège de fibrose empêchant ou rendant difficile le retrait ultérieur de l'implant.

Le but de la présente invention est de proposer un implant de type expansif dont la structure diffère de celles connues par les documents précités, notamment en ce que la stabilisation de l'implant, une fois mis en place, ne risque pas de générer de fibrose empêchant ou rendant difficile le retrait ultérieur de l'implant.

Ce but est parfaitement atteint par l'implant de la présente invention. Il s'agit d'un implant pour la correction du pied plat, destiné à être inséré dans la zone de dimension minimale E donnée d'un sinus tarsien. Cet implant comprend de manière connue d'une part un corps central d'expansion muni de premiers moyens externes de déplacement et d'autre part une enveloppe expansible munie de seconds moyens internes de déplacement aptes à coopérer avec lesdits premiers moyens externes pour le déplacement du corps central à l'intérieur de l'enveloppe et l'expansion de celle-ci.

De manière caractéristique, selon la présente invention,
a) le corps central comporte une partie distale qui est cylindrique, une partie proximale qui est tronconique et une partie intermédiaire qui comporte les moyens externes de déplacement,
b) l'enveloppe externe, de forme cylindrique et dont la face extérieure est exempte de filetage, de rainurage ou d'ailette, comporte :
   - une partie distale qui comporte des fentes longitudinales définissant une pluralité d'ailettes distales,
   - une partie proximale qui comporte des fentes longitudinales définissant une pluralité d'ailettes proximales et
   - une partie intermédiaire qui comporte les moyens internes de déplacement et dont le diamètre D est supérieur à la dimension minimale E, destinée à être disposée dans ladite zone du sinus,
c) la face intérieure des ailettes distales est, sur au moins une partie de sa longueur, inclinée vers l'axe longitudinal de l'enveloppe externe,
d) la face intérieure des ailettes proximales est sensiblement parallèle à l'axe longitudinal de l'enveloppe externe,
   en sorte que, l'implant étant dans le sinus avec la partie intermédiaire de l'enveloppe inséré dans la zone de dimension minimale, le déplacement du corps central à l'intérieur de l'enveloppe provoque le blocage en translation de l'implant du fait de l'expansion des parties proximale et distale de l'enveloppe externe par soulèvement des ailettes proximale et distale.

Dans les documents EP.560.249 et US 2008/0208349A1 c'est l'expansion des deux branches du deuxième corps qui définit l'écartement souhaité entre le talus et le calcanéum. Au contraire, selon la présente invention, cet écartement est défini au niveau de la partie intermédiaire de l'enveloppe externe qui n'est pas expansible. L'expansion des parties distale et proximale, qui intervient après le placement de ladite partie intermédiaire dans la zone de dimension minimale du sinus tarsien, sert à assurer la stabilisation de l'implant dans la position donnée lors de sa mise en place. De ce fait, la partie intermédiaire qui définit l'écartement souhaité entre le talus et le calcanéum n'a pas besoin d'être pourvue, sur sa face extérieure, de rainure ou d'ailette ou de filetage pour assurer cette stabilisation.

Certes, on connaît par les documents US 5.713.904 et DE 40 00 112A1 des dispositifs implantables de type bi-expansif. Mais ces dispositifs sont destinés à des applications très différentes, respectivement pour la fixation dans la colonne vertébrale pour le premier et comme implant intra-oral pour le second. De plus, le dispositif du document US 5.713.904 est implanté par vissage dans un trou préformé dans l'os et comporte donc obligatoirement un filetage sur toute la surface extérieure de son enveloppe externe. Quant au dispositif du document DE 40 00 112A1, son enveloppe externe d'une part comporte des ailettes dans sa partie distale et d'autre part ne comporte pas de partie intermédiaire non expansible, du fait que les fentes délimitant les parties proximale et distale expansibles se prolongent jusqu'au même niveau, ce qui est rendu possible grâce à leur décalage angulaire.

Ainsi, face au problème mentionné ci-dessus et étant donné que les documents connus dans le domaine de la correction du pied plat enseignent de positionner dans la zone de dimension minimale E du sinus du tarse une partie expansible de l'implant, aucun des deux documents US 5.713.904 et DE 40 00 112A1, même s'ils décrivent des dispositifs implantables bi-expansibles, ne peut être pris en considération d'une part parce qu'ils ne concernent pas le domaine de l'invention et d'autre part parce qu'ils ne suggèrent pas la solution apportée par les caractéristiques de la revendication 1 selon lesquelles c'est la partie intermédiaire non expansible, exempte de filetage, rainurage et ailette et dont le diamètre D est supérieur à la dimension minimale E du sinus du tarse, qui est destinée à être disposée dans ladite zone du sinus.

Selon une variante de réalisation, l'implant comporte au moins trois et de préférence au moins quatre ailettes, proximales et distales. Cette disposition particulière permet d'éviter les risques de rupture totale ou partielle de l'ailette, lors du soulèvement de celle-ci, au niveau de la zone de pliage entre l'ailette et la partie intermédiaire.

De préférence chaque ailette comporte une rainure transversale interne à proximité immédiate de la partie intermédiaire. Cette rainure transversale forme une ligne courbe d'épaisseur réduite au niveau de laquelle intervient le pliage de l'ailette.

Selon une variante de réalisation, les angles d'inclinaison α, β respectivement de la face intérieure des ailes distales de l'enveloppe et de la face extérieure de la partie proximale tronconique du corps central, lesdits angles étant mesurés par rapport à l'axe longitudinal de l'implant, sont sensiblement égaux. De préférence ces angles d'inclinaison α et β sont de l'ordre de 20°.

La présente invention sera mieux comprise à la lecture de la description qui va être faite ci-après d'un exemple préféré de réalisation d'un implant pour la correction du pied plat de type bi-expansif, illustré par le dessin annexé dans lequel :
La figure 1 est une vue latérale de la structure osseuse d'un pied gauche laissant apparaître une partie de l'implant positionnée dans le sinus du tarse,
La figure 2 est une représentation en perspective et séparée l'une de l'autre des deux pièces composant l'implant de l'invention à savoir le corps central d'expansion et l'enveloppe externe expansible,
La figure 3 est une vue en perspective de l'implant de la figure 2 avec le corps central introduit dans l'enveloppe externe mais sans qu'il y ait encore d'expansion,
La figure 4 est une représentation en coupe longitudinale de l'implant de la figure 3,
La figure 5 est une représentation en perspective de l'implant de la figure 2 dans laquelle les ailettes proximale et distale sont en expansion,
La figure 6 est une représentation schématique en coupe longitudinale de l'implant de la figure 5,
Les figures 7, 8 et 9 sont des vues éclatées du pied de la figure 1 montrant l'implant dans le sinus du tarse dans la direction générale des flèches respectivement 7, 8 et 9 de la figure 1.

L'implant ou ondoprothèse de la présente invention est destiné à corriger la pathologie dite du pied plat due à un défaut dans l'alignement du talus 2 et du calcanéum 3 provoquant un affaissement de l'arche plantaire. Cette correction s'effectue en introduisant l'implant 1 dans le sinus du tarse 4 ou canal tarsien qui correspond à l'espace libre entre le talus 2 et le calcanéum 3. L'implant 1 est configuré en sorte de rétablir entre le talus 2 et le calcanéum 3 l'écartement et le positionnement adéquat. Pour ce faire l'implant 1 est inséré dans le sinus du tarse 4 dans la zone 4a de dimension minimale E.

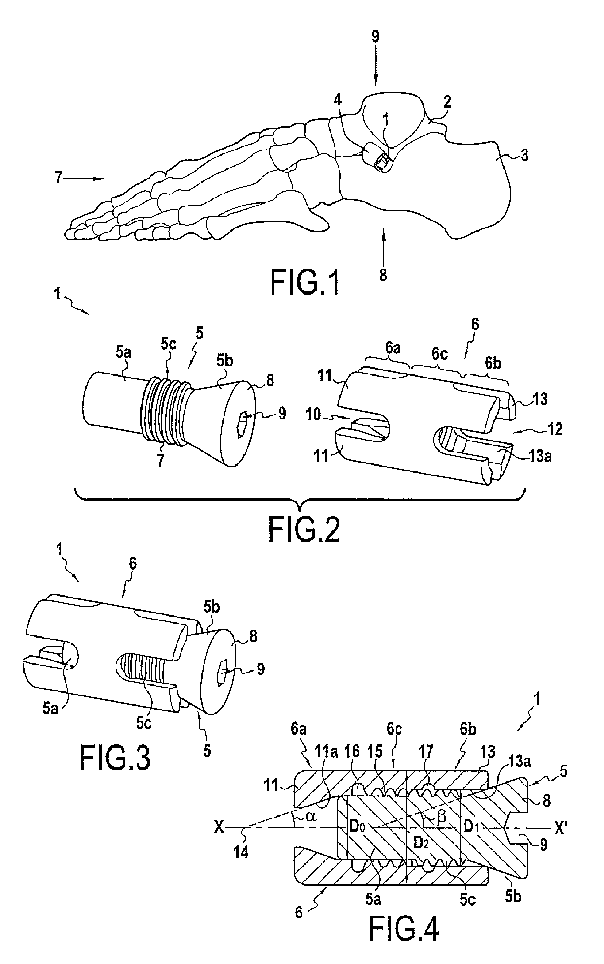

L'implant 1 est composé de deux pièces, à savoir un corps central 5 et une enveloppe externe 6. Ces deux pièces sont représentées, indépendamment l'une de l'autre, sur la figure 2.

Le corps central 5 comporte une partie distale 5a qui est cylindrique, une partie proximale 5b qui est tronconique et une partie intermédiaire 5c qui est également cylindrique mais qui présente un filetage externe 7. Le diamètre de la petite base du tronc de cône de la partie proximale 5b est identique à celui D0 de la partie distale 5a et de la partie intermédiaire 5c hormis bien sûr le filetage externe 7. Dans la face 8 de la partie proximale 5b qui correspond à la grande base du tronc de cône est percé un trou borgne 9 qui dans l'exemple illustré aux figures 1 et 3 a une configuration octogonale. Ce trou borgne 9 est destiné à accueillir la tête d'un outil de vissage.

L'enveloppe externe 6 est une pièce qui a une configuration extérieure cylindrique et qui présente un évidement intérieur dans lequel est amené à se déplacer le corps central 5 lors de la mise en place de l'implant 1 comme cela sera expliqué ci-après.

L'enveloppe externe 6 comporte, sur sa longueur, trois parties fonctionnellement distinctes, à savoir une partie distale 6a, une partie proximale 6b et une partie intermédiaire 6c.

La partie distale 6a comporte des fentes longitudinales 10 qui définissent entre elles une pluralité d'ailettes distales 11 ; de même la partie proximale 6b comporte des fentes longitudinales 12 qui définissent entre elles une pluralité d'ailettes proximales 13. Dans l'exemple détaillé qui est illustré à la figure 2, il y a quatre fentes longitudinales 10, 12, respectivement dans les parties distales 6a et 6b de l'enveloppe externe 6, lesquelles définissent quatre ailettes distales 10 et quatre ailettes proximales 13. Dans cet exemple, les fentes longitudinales 10, 12 sont alignées et de même largeur de sorte que les ailettes distale 11 et proximale 13 s'étendent selon le même arc de cercle et sont également alignées.

Comme cela est visible sur la figure 4, la face intérieure 11a de chaque ailette distale 11 est, sur au moins une partie de sa longueur, inclinée vers l'axe longitudinal XX' d'un angle α. Le point d'intersection 14 de l'axe longitudinale XX' avec la direction générale de la face intérieure 11a de l'ailette distale 11 au repos se trouve à l'extérieur de l'enveloppe externe 6. La face intérieure 13a de chaque ailette proximale 13 est sensiblement parallèle à l'axe longitudinal XX'. Le diamètre intérieur D1 de l'enveloppe externe 6, au niveau des ailettes proximales 13, est légèrement supérieur au diamètre de la partie intermédiaire 5c du corps central 1, incluant le filetage externe 7.

La partie intermédiaire 6c de l'enveloppe externe 6 comporte un filetage interne 15 qui est apte à coopérer avec le filetage externe 7 de la partie intermédiaire 5c du corps central 5. Le diamètre intérieur de la partie intermédiaire 6c, au niveau du filetage 15 est légèrement supérieur au diamètre D0 de la partie distale 5a du corps central 5.

On a représenté sur la figure 4 l'implant 1 avec le corps central 5 qui est introduit dans l'évidement intérieur de l'enveloppe externe 6 alors que le filetage externe 7 du corps central est juste engagé dans le filetage interne 15 de l'enveloppe externe. Dans cette configuration, le corps central 5 est déjà solidarisé à l'enveloppe externe 6 mais les ailettes distale 11 et proximale 13 sont encore au repos. Ceci permet au praticien de manipuler l'implant comme s'il s'agissait d'une seule pièce pour son introduction dans le sinus du tarse.

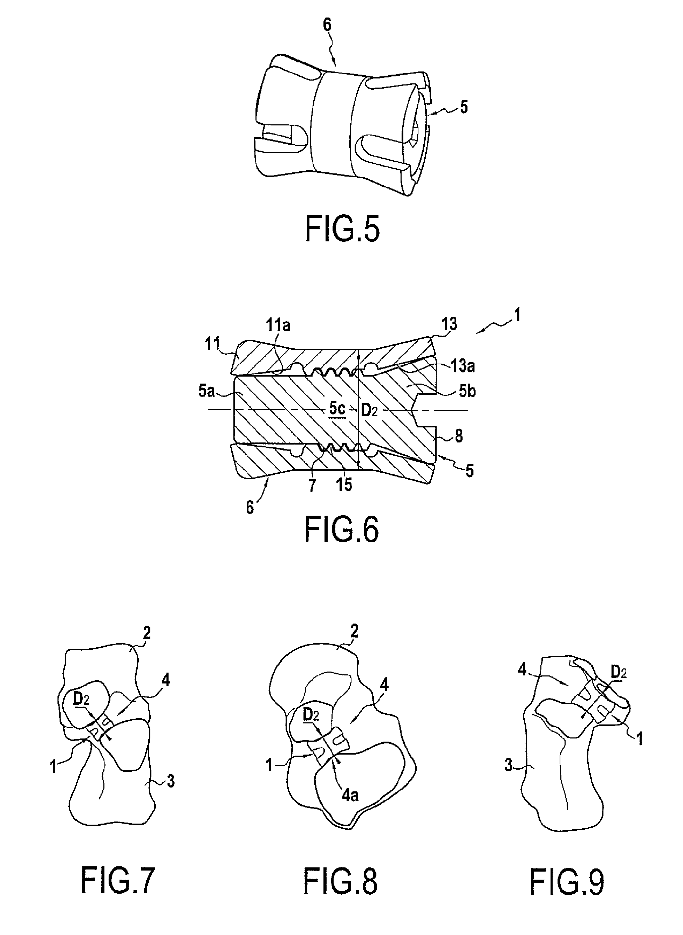

C'est le diamètre externe D2 de la partie intermédiaire 6c de l'enveloppe externe 6 qui est déterminant pour la correction souhaitée puisque c'est cette partie intermédiaire 6c qui est destinée à être insérée dans la zone de dimension minimale E du sinus du tarse 4. Une fois inséré dans ladite zone, à l'aide d'un gabarit, le praticien va assurer la stabilisation de l'implant 1 en réalisant l'expansion des parties distale 6a et proximale 6b de l'enveloppe externe 6. Pour cela il introduit la tête de l'outil de vissage dans le trou borgne 9 et fait pivoter le corps central 5 dans le sens des aiguilles d'une montre. Ce vissage entraîne le déplacement du corps central 5 vers l'intérieur de l'enveloppe externe 6 grâce à la coopération des filetages externe 7 et interne 15. Lors de ce déplacement, comme cela apparaît sur la figure 6 se produit dans le même temps le soulèvement des ailettes distales 11 et des ailettes proximales 13. Plus précisément l'extrémité de la partie distale 5a du corps central 5 vient, lors de ce déplacement, en appui sur la partie inclinée de la face intérieure 11a, ce qui a pour effet de repousser chaque ailette distale 11. Le même effet mais inverse est observé en ce qui concerne les ailettes proximales 13. C'est la face inclinée de la partie proximale 5b du corps central 5 qui vient, lors de ce déplacement, en appui sur la face intérieure 13a, repoussant chaque ailette 13. La face inclinée de la partie proximale tronconique 5b du corps central 5 forme un angle β par rapport à l'axe longitudinal dudit corps central 5, qui est confondu avec l'axe XX' lorsque ledit corps central 5 est introduit à l'intérieur de l'enveloppe externe 6. Dans l'exemple illustré à la figure 4, les angles d'inclinaison α et β sont du même ordre, faisant de l'ordre de 20°. Cette disposition permet aux ailettes distale 11 et proximale 13 d'être sensiblement en contact au moins partiel avec certaines zones du talus 2 et du calcanéum 3 lors de l'expansion desdites ailettes après insertion de l'implant 1 comme cela apparaît sur les figures 7, 8 et 9.

Le matériau dans lequel est formée l'enveloppe externe 6 doit présenter une certaine capacité de déformation pour permettre le soulèvement des ailettes 11, 13. De préférence l'enveloppe externe 6 est formée à partir d'un polymère biocompatible. Pour faciliter le soulèvement des ailettes 11, 13, il est prévu, comme cela apparaît sur les figures 4 et 6, des rainures transversales internes, formées en creux, à proximité immédiate de la partie intermédiaire 6c respectivement dans la partie distale 6a et la partie proximale 6b. Ces rainures 16, 17 diminuent localement l'épaisseur des ailettes 11, 13, formant en quelque sorte une ligne de pliage d'épaisseur réduite, facilitant le soulèvement des ailettes 11, 13.

L'implant 1 n'est normalement pas destiné à être maintenu en place de manière définitive. Il est le plus souvent retiré une fois qu'a pu être constaté de manière durable le redressement de la voûte plantaire. Pour ce retrait, le praticien introduit l'outil de vissage dans le trou borgne 9, fait pivoter le corps central 5 dans le sens contraire des aiguilles d'une montre jusqu'à ce que les ailettes distale 11 et proximale 13 reviennent dans la position de repos illustrée à la figure 4. Dans cette position, le corps central 5 est encore solidarisé à l'enveloppe externe 6 du fait de l'engagement partiel des filetages 7 et 15. Ainsi il suffit au praticien, pour effectuer le retrait de l'implant, de saisir à l'aide d'un outil adapté l'extrémité de la partie proximale 5b du corps central 5 dépassant de l'enveloppe externe 6 et d'effectuer une traction sur celle-ci.

Il est à noter que la surface extérieure de l'enveloppe externe 6 est, dans l'exemple illustré, totalement dépourvue de rainurage ou autre saillie facilitant l'accrochage de fibrose tissulaire.

Pour éviter le retrait de l'implant, il est aussi possible de former le corps central 5 et l'enveloppe externe 6 dans un matériau résorbable, la résorption dudit matériau permettant de libérer progressivement le sinus du tarse de l'implant 1.

Le diamètre D2 de la partie intermédiaire 6c de l'enveloppe externe est choisi en fonction du patient dont il convient de corriger la pathologie du pied plat. Elle est généralement comprise entre 5 et 11 mm.

Par exemple pour un diamètre D2 de 7 mm, l'enveloppe externe 6 avait une longueur totale de 15 mm répartie à raison de 5,5 mm pour la partie distale 6a, 5 mm pour la partie proximale 5 et 4,5 mm pour la partie intermédiaire 6c, étant entendu que les rainurages internes font partie intégrante des parties proximale et distale.

La présente invention n'est pas limitée au mode de réalisation qui a été décrit ci-dessus à titre d'exemple non limitatif. En particulier les moyens interne et externe de déplacement ne sont pas nécessairement des filetages interne et externe, aptes à coopérer pour le déplacement du corps central à l'intérieur de l'enveloppe externe par vissage.

Il peut s'agir de crans notamment formés sur l'extérieur du corps central, lesdits crans étant déformables pour pouvoir être déplacés par impaction, à l'intérieur de l'enveloppe externe jusqu'à rentrer dans des logements en creux dans l'enveloppe externe. Ce mode de réalisation ne permet cependant pas le retrait facile de l'implant par simple dévissage et traction sur l'extrémité du corps central comme expliqué ci-dessus.

## Revendications

1. Implant pour la correction du pied plat, destiné à être inséré dans la zone de dimension minimale E donnée d'un sinus du tarse (4), comprenant d'une part un corps central d'expansion muni de premiers moyens externes de déplacement et d'autre part une enveloppe expansible munie de seconds moyens internes de déplacement aptes à coopérer avec lesdits premiers moyens externes pour le déplacement du corps central à l'intérieur de l'enveloppe et l'expansion de celle-ci, **caractérisé en ce que** :
- le corps central (5) comporte une partie distale (5a) qui est cylindrique, une partie proximale (5b) qui est tronconique et une partie intermédiaire (5c) qui comporte les moyens externes de déplacement (7),
- l'enveloppe externe (6), de forme cylindrique et dont la face extérieure est exempte de filetage, de rainurage ou d'ailette, comporte une partie distale (6a) qui comporte des fentes longitudinales (10) définissant une pluralité d'ailettes distales (11), une partie proximale (6b) qui comporte des fentes longitudinales (12) définissant une pluralité d'ailettes proximales (13) et une partie intermédiaire (6c) qui comporte les moyens internes de déplacement (15), dont le diamètre (D2) est supérieur à la dimension minimale E et qui est destinée à être disposée dans ladite zone du sinus,
- la face intérieure (11a) des ailettes distales (11) est, sur au moins une partie de sa longueur, inclinée vers l'axe longitudinal (XX') de l'enveloppe externe (6),
- la face intérieure (13a) des ailettes proximales (13) est sensiblement parallèle à l'axe longitudinal (XX') de l'enveloppe externe (6),
en sorte que, l'implant (1) étant dans le sinus (4) avec la partie intermédiaire (6c) de l'enveloppe externe (6) inséré dans la zone de dimension minimale E, le déplacement du corps central (5) à l'intérieur de l'enveloppe (6) provoque le blocage en translation de l'implant (1) du fait de l'expansion des parties proximale (6a) et distale (6b) de l'enveloppe externe (6) par soulèvement des ailettes proximales (11) et distales (13).

2. Implant selon la revendication 1 **caractérisé en ce que** les moyens internes de déplacement consistent en un filetage interne (15) formé dans la partie intermédiaire (6c) de l'enveloppe externe (6) et **en ce que** les moyens externes de déplacement comprennent un filetage externe (7) formé sur la partie intermédiaire (5c) du corps central (5) et un moyen externe de vissage.

3. Implant selon la revendication 1 **caractérisé en ce que** les moyens internes et externes de déplacement sont formés de crans et de logements, permettant le déplacement du corps central à l'intérieur de l'enveloppe par impaction.

4. Implant selon l'une des revendications 1 à 3 **caractérisé en ce que** le diamètre (D2) de la partie intermédiaire de l'enveloppe est de l'ordre de 5 à 11 mm.

5. Implant selon l'une des revendications 1 à 4 **caractérisé en ce que** chaque ailette proximale (13) est dans l'alignement d'une ailette distale (11).

6. Implant selon l'une des revendications 1 à 5 **caractérisé en ce qu'**il comporte au moins trois et de préférence au moins quatre ailettes proximales (13) et ailettes distales (13).

7. Implant selon l'une des revendications 1 à 6 **caractérisé en ce que** chaque ailette (11,13) comporte une rainure transversale interne (16,17) à proximité immédiate de la partie intermédiaire (6c).

8. Implant selon l'une des revendications 2 à 7 **caractérisé en ce que** le moyen externe de vissage comporte un trou borgne (9), notamment de section polygonale, formé dans la grande base de la partie proximale (5b) tronconique du corps central (5), dans lequel peut pénétrer de manière ajustée la tête d'un outil de vissage.

9. Implant selon l'une des revendications 1 à 8 **caractérisé en ce que**, mesurés par rapport à son axe longitudinal (XX'), les angles d'inclinaison α,β respectivement de la face intérieure (11a) des ailettes distales (11) de l'enveloppe (6) et de la face inclinée de la partie proximale (5b) tronconique du corps central (5) sont sensiblement égaux.

10. Implant selon la revendication 9 **caractérisé en ce que** les angles d'inclinaison α et β sont de l'ordre de 20°.
